(19) Europäisches Patentamt
European Patent Office
Office européen des brevets

(11) **EP 4 657 458 A1**

(12) **EUROPEAN PATENT APPLICATION**

(43) Date of publication:
**03.12.2025 Bulletin 2025/49**

(21) Application number: **24179014.6**

(22) Date of filing: **30.05.2024**

(51) International Patent Classification (IPC):
**G16H 50/20** (2018.01)     **A61B 5/00** (2006.01)
**G16H 50/30** (2018.01)     **G16H 50/70** (2018.01)

(52) Cooperative Patent Classification (CPC):
**G16H 50/20; A61B 5/72; G16H 50/30; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **CONROY, Bryan**
  **Eindhoven (NL)**
• **DA SILVA, Ikaro Garcia Araujo**
  **Eindhoven (NL)**
• **SALVATI, Emmanuele**
  **5656AG Eindhoven (NL)**
• **DAMIANO, Robert John**
  **Eindhoven (NL)**

(74) Representative: **Philips Intellectual Property & Standards**
**High Tech Campus 52**
**5656 AG Eindhoven (NL)**

(54) **DEVICE AND METHOD FOR ESTIMATING A PROBABILITY OF A HEALTH STATE OF A PATIENT**

(57)     The present invention relates to device for estimating a probability of a health state of a patient, particularly a probability of an infection of the patient, the device comprising an input unit configured to obtain physiological data of the patient, the physiological data comprising information about a plurality of time series of physiological parameters of the patient, a processor configured to extract the plurality of time series from the physiological data and to apply a machine learning algorithm on the plurality of time series to estimate the probability of the health state of the patient, and an output unit configured to provide the probability of the health state, wherein the machine learning algorithm is based on a generalized additive model, in which results of an application of a learned non-linear univariate function on the parameter time series are summed up and the probability of the health state is obtained by applying the standard sigmoid function on said sum.

FIG.1

EP 4 657 458 A1

**Description**

FIELD OF THE INVENTION

This invention was made with Government support under contract no.HDTRA121C0006 awarded by Defense Threat Reduction Agency. The Government has certain rights in this invention.

The present invention relates to a device and method for estimating a probability of a health state of a patient. Furthermore, the present invention relates to a training device and training method for training a generalized additive model to be used for estimating a probability of a health state of a patient.

BACKGROUND OF THE INVENTION

Developing predictive models of patient deterioration and patient state in healthcare is an important and challenging problem. In many applications it is important for the developed models to maintain a level of interpretability, which enables trust to be established between the model output and clinicians, care-givers, and individuals. Despite this goal, the patient data acquired from (professional) patient monitoring devices and/or commercial off-the-shelf (COTS) wearable devices generally comprise complicated, high-dimensional time-series that make it challenging for traditional and interpretable machine learning models to be made effective.

In fact, standard techniques to train temporal-based models would use neural networks or other deep learning techniques, however these suffer from a lack of interpretability. On the other hand, decision tree models suffer from not being smooth with respect to the underlying feature space; as a result, deploying them as time-series classification models may lead to undesirable jumpiness in the predictive model score over time.

SUMMARY OF THE INVENTION

It is an object of the present invention to provide a device and method allowing to estimate a probability of a health state of a patient in an effective, reliable and easily interpretable manner. It is another object to provide a training device and training method allowing to train the machine learning model(s) used by the device and method for estimating a probability of a health state of a patient und thus to support said device and method in estimating the probability of the health state in an effective, reliable and easily interpretable manner.

In a first aspect of the present invention a device for estimating a probability of a health state of a patient, particularly a probability of an infection of the patient, is presented, the device comprising

an input unit configured to obtain physiological data of the patient, the physiological data comprising information about a plurality of time series of physiological parameters of the patient,
a processor configured to extract the plurality of time series from the physiological data and to apply a machine learning algorithm on the plurality of time series to estimate the probability of the health state of the patient, and
an output unit configured to provide the probability of the health state,
wherein the machine learning algorithm is based on a generalized additive model, in which results of an application of a learned non-linear univariate function on the parameter time series are summed up and the probability of the health state is obtained by applying the standard sigmoid function on said sum.

In another aspect of the present invention a training device for training a generalized additive model is presented, the training device comprising

an input unit configured to obtain training data comprising example parameter time series, wherein each example parameter time series is labelled with a label indicating a health status,
a storage unit configured to store the generalized additive model, wherein in the generalized additive model the results of an application of a non-linear univariate function on the parameter time series are summed up for use in estimating a probability of the health status of a patient,
a learning unit configured to learn the non-linear univariate functions by using the training data, and
an output unit configured to provide the generalized additive model with the learned non-linear univariate functions.

In a further aspect of the present invention a system for estimating a probability of a health state of a patient is presented, the system comprising a device for estimating a probability of a health state of a patient and a rendering unit configured to render visual and/or audible information in accordance with the estimated probability of the health state of the patient.

In yet further aspects of the present invention, there are provided corresponding methods, computer programs which comprises program code means for causing a computer to perform the steps of the methods disclosed herein when said computer programs are carried out on a computer as well as non-transitory computer-readable recording media that store therein the respective computer program products, which, when executed by a processor, causes the methods disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to provide a device which is capable of indicating early signs of illness (particularly infection exposure) from patient monitoring data in order to provide additional time in early stages of illness for proactive action. The generalized additive model used in the device for estimating a probability of a health state is implemented as a classification model (which preferably runs in real-time) to predict/estimate the probability of the health state given the patient's current and/or historical physiological (monitoring) data. The invention proposes an algorithm having high expressiveness of the predictive model while maintaining interpretability.

As mentioned above the device for estimating a probability of a health state is configured to apply a machine learning algorithm which is based on a generalized additive model (GAM). For training the generalized additive model a training device is presented. For training, training data are obtained, the training data comprising example parameter time series, wherein each example parameter time series is labelled with a label indicating a health status. More particularly, there is obtained raw input physiological data as a d-dimensional vector time-series

$$x(t) = [x_1(t), x_2(t), ..., x_d(t)]$$

in which each dimension may represent a different physiological input. Accordingly, $x_1(t)$ may be a temperature time-series, $x_2(t)$ may be a heart rate time-series etc. An initial transformation may be applied to convert the raw data into a new p-dimensional vector time-series

$$z(t) = [z_1(t), z_2(t), ..., z_p(t)]$$

of derived features, which may include statistics on the physiological measurements (max, min, mean, median, etc.), baseline corrected features (e.g., via personalized z-scoring), and trend features that capture deviations from a previous level. In some applications, these derived features may depend on additional context information that may be available (e.g., the activity level or sleep patterns of the patient). Furthermore, the transformed $z(t)$ may summarize the raw physiological data over certain contextual periods, such as sleep periods or periods of rest. In these cases, $z(t)$ may be sampled at a much lower temporal resolution at times that correspond to the end of these contextual periods, such as when the patient wakes up each day. Since these derived features can be applied to each of the original d physiological inputs, it is possible that p >> d.

In general, for training either the original raw input physiological data (as a d-dimensional vector time-series) may be used, or there may be used the time series of derived features.

During training, it is assumed that each time-series $z(t)$ (or $x(t)$) is associated with a label, the label (y) ultimately being the target for the predictive model, i.e. a health state of the patient (e.g., infected or healthy). In an ideal scenario, a precise timing on when a patient transitions between states is desired, however this is not often achievable in practice, especially when the label state is derived from surveys or diagnostic test results. Moreover, the timing of when a physiological pattern expresses itself relative to a state transition may vary based on the patient and other factors. This implies that there is only a coarse temporal representation of the patient state available. As a result, it is assumed that a single label (state) is associated with whole time segments of $z(t)$. As an example, a label may represent whether or not a diagnostic test was positive, and $z(t)$ is the corresponding segment of data from the week leading up to the test. Therefore, the training data consist of $n$ labeled time segments

$$\left( (z^{(1)}(t), y^{(1)}(t)), ..., (z^{(n)}(t), y^{(n)}(t)) \right)$$

where each $z^{(i)}(t)$ is a segmented time-series and $y^{(i)}(t)$ is its corresponding label, wherein e.g., $y^{(i)} = 1$ if the example corresponds to an infection case, and $y^{(i)} = 0$ if the example corresponds to a healthy (non-infection) case. Without loss of generality, it is assumed that each $z^{(i)}(t)$ segment is of length $T$, though the method generalizes to cases where the length of each segment varies. Note that the length $T$ should reflect the timing uncertainty of the physiological patterns that emerge as a result of the clinical event and is very much informed by the specifics of the use case. It is also possible to treat this selection as a hyper-parameter to be tuned. The final goal is to train the model used so that it predicts $y^{(i)}(t)$ from each $z^{(i)}(t)$.

As mentioned above, the predictive model used is a generalized additive model, which takes as input the p-dimensional vector of derived features (z in the above), and outputs a predictive score *s* (note that in the following the time argument is dropped to simplify notation, but in practice both z and s are time-series):

$$s(z) = \sum_{j=1}^{p} s_j(z_j)$$

In the above formula $s_j$ is a non-linear univariate function that operates on a single derived feature (i.e. single times series (vector) of derived parameters), wherein $s_0$ is an offset value, which is set to zero here in this case. In general, $s_0$ may be non-zero. In the above formula, the score *s* can be regarded as a risk score or risk function indicating the risk whether a patient, to which the features *z* are assigned, exhibits a particular health state, for example an infection. Hence, *s* reflects the relationship between *z* and the label *y* which is to be predicted by the model.

As can be seen, GAMs are a class of machine learning models where the prediction may be expressed as the linear sum of the predictor functions of individual inputs.

To learn the s-functions boosting techniques are used. There is used a loss function to assess how well the model is doing at predicting the labels *y*. The model is trained until the loss function falls below a predetermined value.

The probability of infection of a patient is given by $\sigma(s(z))$ where $\sigma$ is the standard sigmoid function. The standard sigmoid function, also known as the logistic sigmoid function, is commonly used in machine learning for binary classification tasks because of its properties that make it suitable for representing probabilities.

Given a trained GAM, the device for estimating (i.e. predicting) the probability of a health state of the patient is capable of precisely predicting the probability of the health state. For instance, a device using a GAM that was trained with training data comprising temperature time-series, in which the temperatures with labels 0 or 1, the label 0 indicating that the patient has no fever and the label 1 indicating that the patient has fever, may provide a precise probability of a patient having fever if a temperature time series measured from the patient was input. Contrary to decision tree model, for example, the output of the GAM, i.e. the probability, is smooth, which is due to the fact that the *s*-functions are smooth.

Preferably, the device for estimating a probability of a health state of a patient, or more particularly its processor, is configured to apply a machine learning algorithm based on the generalized additive model trained and provided by the training device for training the generalized additive model as presented herein.

In an embodiment of the device, in the generalized additive model the sum of the learned non-linear functions on the parameter time series is supplemented by an interaction component, the interaction component comprising a learned bivariate function of two of the learned non-linear univariate functions of the parameter time series, wherein the probability of the health state is obtained by applying the standard sigmoid function on a sum of the sum of the learned non-linear univariate functions and the learned bivariate function.

In other words, in this embodiment the device uses a GAM which is augmented by an interaction component (i.e. "feature interaction term") $a(s_j, s_k)$, wherein *a* is a learned bivariate function of $s_j$ and $s_k$, $s_j$ representing a learned non-linear univariate function of a first feature $z_j$ and $s_k$ representing a learned non-linear univariate function of a second (different) feature $z_k$. For example, $s_j$ could be a learned non-linear function of a temperature and $s_k$ could be a learned non-linear univariate function of a heart rate. Preferably, both features are measured at the same time (and are features of the same patient). Hence, in the embodiment the device uses a GAM of the following type:

$$s^{new}(z) = s^{old}(z) + a\big(s_j(z), s_k(z)\big)$$

where $s^{old}(z)$ represents the original GAM, i.e. the GAM without feature interaction terms, and where $s_j$ and $s_k$ are selected feature interaction terms and *a* is a learned aggregation function that governs the interaction between $s_j$ and $s_k$. In the above example, the bivariate function *a* would thus describe a feature interaction between the features temperature and heart rate of the patient, *a* is a function which has been learned using machine learning tools. In the example using the features temperature and heart rate it can be expected that the function *a* describes a conjunctive aggregation as, according to Liebermeister's rule there is an increment ratio between an adult individual's cardiac frequency and temperature when in fever. According to this (rough) rule, each Celsius grade of <u>body temperature</u> increment roughly corresponds to an 8 beats per minute increase in cardiac frequency.

The purpose of augmenting the GAM in this manner is to increase the expressiveness (and therefore the predictive power), while still maintaining model interpretability.

In another embodiment of the device, the interaction component comprises a sum of learned bivariate functions of two of the learned non-linear univariate functions of the parameter time series, wherein the probability of the health state is obtained by applying the standard sigmoid function on a sum of the sum of the learned non-linear univariate functions and the sum of the learned bivariate functions.

In other words, it may be assumed that

$$s^{new}(z) = s^{(0)}(z) + \sum_{r=1}^{R} a^{(r)}\left(s_{j_r}(z), s_{k_r}(z)\right).$$

Accordingly, there are $R$ interaction terms taken into account in this embodiment.

In another embodiment, the device further comprises an explanation unit configured to identify parameters driving the probability by ordering the learned non-linear univariate functions of the parameter time series by ranking the features by their individual risk scores.

In yet another embodiment of the device, if the sum of the generalized additive model is supplemented by the interaction component, the explanation unit is further configured to analyze the bivariate function (or bivariate functions) to assess whether the corresponding interaction component is conjunctive, disjunctive or compensatory. Furthermore, the explanation unit may be configured to order the learned bivariate functions by rank to identify interactions driving the probability of the health state.

Preferably, the learned bivariate function(s) may be parametrized as a uninorm aggregation function:

$$a^{(r)}(x, y) = \left(f^{(r)} \circ \sigma\right)^{(-1)} \left(\left(f^{(r)} \circ \sigma\right)(x) + \left(f^{(r)} \circ \sigma\right)(y)\right)$$

where $f^{(r)} \circ \sigma$ is the additive generator of the uninorm, $\sigma$ is the standard sigmoid function and $f^{(r)}$ is a monotonically increasing function on [0,1]. The benefit of modeling the aggregation function in this way is that the uninorm can flexibly learn many forms of aggregation, including conjunctive (e.g., product), disjunctive (e.g., probabilistic sum), and compensatory (e.g., average) and that the form of aggregation induced by the uninorm can readily be (analyzed and) interpreted by the explanation unit of the device.

In fact, the explanation unit may analyze the $a$-functions, particularly $f^{(r)}$ to extract whether the underlying interactions are conjunctive, disjunctive or compensatory. For example, if $f^{(r)}$ comprises a product the interaction of the underlying interaction parameters can be interpreted to be conjunctive.

In a further embodiment, the device is configured to provide the probability of the health state in real time.

Hence, as soon as physiological data of the patient are available, the device for estimating the probability of a health state may be provided with said data and may essentially immediately provide the probability of the health state. For example, the device may provide a value for $\sigma(s(z))$ (where $s(z)$ is given with or without involving interaction components) as soon as one or more time series of measured features (or features derived from the measured features) of the patient are available for the device for estimating a probability of a health state.

In an embodiment of the training device, the learning unit is configured to supplement the sum of the learned non-linear functions on the parameter time series by an interaction component, the interaction component comprising a bivariate function of two of the learned non-linear univariate functions of the parameter time series, particularly a sum of bivariate functions of two of the learned non-linear univariate functions, and to learn the bivariate function or bivariate functions, wherein the output unit is configured to provide the generalized additive model with the learned bivariate function or bivariate functions.

To this end a (boosting-based) training procedure (of the training device) is used to augment the model (without interaction components) with feature interaction terms.

For the (boosting-based) training the same training data

$$\left((z^{(1)}(t), y^{(1)}(t)), \dots, (z^{(n)}(t), y^{(n)}(t))\right)$$

as used for training the original GAM may be applied. Starting from an initial model $s^{old}(z)$ (where $s^{old}(z)$ is the original GAM, i.e. the GAM without interaction component), the boosting model may incorporate a feature interaction term, such that:

$$s^{new}(z) = s^{old}(z) + a\left(s_j, s_k\right)$$

where $s_j$ and $s_k$ are selected feature interaction terms and $a$ is a learned aggregation function that governs the interaction between $s_j$ and $s_k$, i.e. the interaction between different features of the patient (as derived from measurements). Thus, the goal is to select the terms $s_j$ and $s_k$, as well as the aggregation function $a$. To this end, $a$ may be parametrized in a suitable manner.

In an embodiment of the training device the learning unit is configured to parametrize the bivariate function(s) as uninorm function(s).

In another embodiment of the training device the learning unit is configured to learn the bivariate functions iteratively.

Starting from an initial model at $r = 0$, $s^0(z) = s(z) = s^{old}(z)$. Using a boosting model, feature interaction terms can be iteratively incorporated for iterations r = 1,2, ..., *R:*

$$s^{(r+1)}(z) = s^{(r)}(z) + a^{(r)}\left(s_{j_r}(z), s_{k_r}(z)\right)$$

where $s_j$ and $s_k$ are the selected feature interaction terms for that iteration and $a^{(r)}$ is to be learned for that iteration. The goal at each iteration is to select the feature interaction terms $s_{j_r}$ and $s_{k_r}$ as well as the aggregation function $a^{(r)}$. To this end, $a^{(r)}$ may preferably be parameterized as a uninorm aggregation function

$$a^{(r)}(x, y) = \left(f^{(r)} \circ \sigma\right)^{(-1)} \left(\left(f^{(r)} \circ \sigma\right)(x) + \left(f^{(r)} \circ \sigma\right)(y)\right)$$

where $f^{(r)} \circ \sigma$ is the additive generator of the uninorm, $\sigma$ is the standard sigmoid function and $f^{(r)}$ is a monotonically increasing function on [0,1]. Further to the benefits (of such a parametrization) mentioned above, provided that the risk functions $s_j$ and $s_k$ from the underlying GAM are smooth (which is the case), another benefit of this parameterization is that the feature interaction, i.e. the function $a$, will also be smooth.

To learn the interaction terms and aggregation function, functional gradient descent on a loss function (e.g., binary cross-entropy loss) is used, averaged over the training data $((z^{(1)}(t), y^{(1)}(t)), ..., (z^{(n)}(t), y^{(n)}(t)))$. The additive generator $f^{(r)}$ can either be parameterized as an additive mixture of template generators, or it may be parameterized in terms of its gradient (since it is monotonically increasing, its gradient must be greater than zero everywhere). At the end of R iterations (where R is a hyperparameter that can be tuned via cross-validation), the model is a mixture of the original GAM and a set of R feature interactions:

$$s^{new}(z) = s^{(R)}(z) = s^{(0)}(z) + \sum_{r=1}^{R} a^{(r)}\left(s_{j_r}(z), s_{k_r}(z)\right).$$

with $s^{(0)}(z)$ representing the original GAM model. The final probability of infection exposure is $\sigma(s^{(R)}(z))$, where $\sigma$ is the standard sigmoid function.

In an embodiment of the training device, in each iteration step the learning unit is configured to learn the bivariate function using boosting techniques. In a first step of the iteration the functions $s_k$ and $s_j$ are kept fix and $a$ is learned. Again, in learning the *a*-function the loss of the loss function applied should fall below a predetermined value to be sure to have a classifier working well. In a next step of the iteration another *a*-function, i.e. another interaction of *s*-functions is learned in the same manner and so on.

In general, the bivariate function could also be applied on non-univariate function to get higher interactions, such as a trivariate function. This would increase the predictive power of the model even further. However, interpretability might get lost at some point.

The device for estimating a probability of a health state of a patient and the training device for training the generalized additive model may be combined in a single device. In other words, the device for estimating a probability of a health state of a patient may comprise the (features of the) training device (or vice versa).

Similarly, the corresponding methods may be combined, the combined method comprising

obtaining training data comprising example parameter time series, wherein each example parameter time series is labelled with a label indicating a health state,
storing the generalized additive model, wherein in the generalized additive model the results of an application of a non-linear univariate function on a parameter time series are summed up for use in estimating a probability of a health status of a patient,
learning the non-linear univariate functions by using the training data, and
providing the generalized additive model with the learned non-linear univariate functions,
obtaining physiological data of the patient, the physiological data comprising information about a plurality of time series of physiological parameters of the patient,
extracting the plurality of time series from the physiological data and applying a machine learning algorithm on the plurality of time series to estimate the probability of the health state of the patient, and
providing the probability of the health state,

wherein the machine learning algorithm is based on the generalized additive model with the learned non-linear univariate functions.

In an embodiment of the system for estimating a probability of a health state of a patient (the system comprising the device for estimating a probability of a health state of a patient and a rendering unit configured to render visual and/or audible information in accordance with the estimated probability of the health state of the patient), system further comprises one or more of

a training device for training the generalized additive model (as presented herein),
a user interface, wherein the user interface is configured to obtain user input, particularly physiological data of the patient, the physiological data comprising information about a plurality of time series of physiological parameters of the patient, and/or a plurality of time series extracted from physiological data of the patient;
a database comprising physiological data of the patient, the physiological data comprising information about a plurality of time series of physiological parameters of the patient, and/or comprising a plurality of time series extracted from physiological data of the patient; and
one or more patient sensors configured to measure (as physiological data; the physiological data comprising information about a plurality of time series of physiological parameters of the patient) one or more physiological parameters of the patient.

The steps of the methods presented herein may be carried out by the device for estimating a probability of a health state of a patient or the training device presented, respectively. The method may e.g. be implemented as computer program running on a computer or processing unit.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a system according to the present invention.
Fig. 2 shows a schematic diagram of an embodiment of a device for estimating a probability of a health state of a patient according to the present invention.
Fig. 3 shows a schematic diagram of an embodiment of a training device for training a generalized additive model according to the present invention.
Fig. 4 shows a schematic diagram of a second embodiment of a system according to the present invention.

DETAILED DESCRIPTION OF EMBODIMENTS

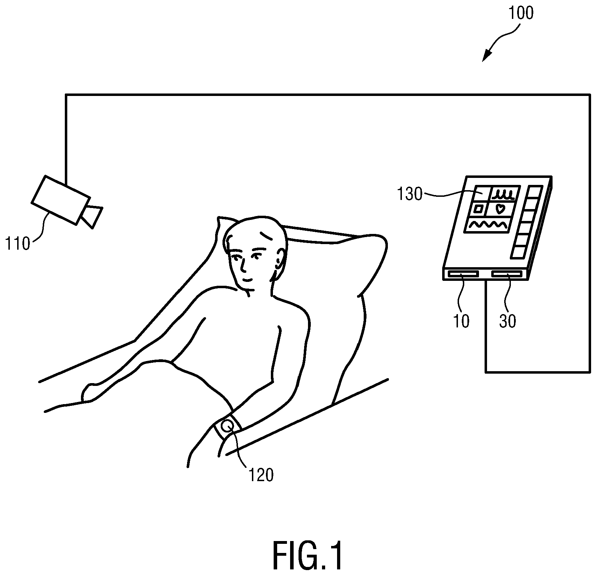

Fig. 1 shows a schematic diagram of a first embodiment of a system according to the present invention. The system 100 comprises a device 10 for estimating a probability of a health state of a patient, a rendering unit 130, and, as patient sensors, a camera unit 110 and a photoplethysmographic(PPG)-sensor unit 120. Optionally, the system may further comprise a training device 30 for training the generalized additive model used in the device 10.
In the shown embodiment, the camera 110 is configured to monitor the patient and to obtain image data of the patient, the image data comprising physiological data of the patient, particularly information about a time series of a physiological parameters of the patient. In particular, the image data may comprise information about the respiration rate of the patient, particularly a respiration rate time series.
The PPG-sensor unit 120 is configured to measure a heart rate, particularly a heart rate time series, at the wrist of the patient and to provide corresponding heart rate data.
The image data of the camera unit 110 and the heart rate data of the PPG-sensor unit 120 are then obtained by the device 10, which then extracts a respiration rate time series and a heart rate time series from said data and further derives other parameter time series from said times series, which are then used by a machine learning algorithm using a GAM to estimate the probability of the health state of the patient.
The rendering unit 130 then renders the probability of the health state estimated by the device for estimating a probability of a health state of a patient as visual information. Accordingly, in this embodiment, the rendering unit 130 is implemented as a display.
In general, the rendering unit 130 may be any means that outputs the estimated probability of the health state in visual or audible form, e.g. in text form, as image or diagram, as sound or spoken words, etc. For instance, the rendering unit may be a display, a loudspeaker, a touchscreen, a computer monitor, the screen of a smartphone or tablet, etc.
In this embodiment of the system 100, the device 10 and the rendering unit 130 are arranged in the same housing, particularly a housing of a medical device. The training device 30, if provided, may likewise be arrange in said housing.

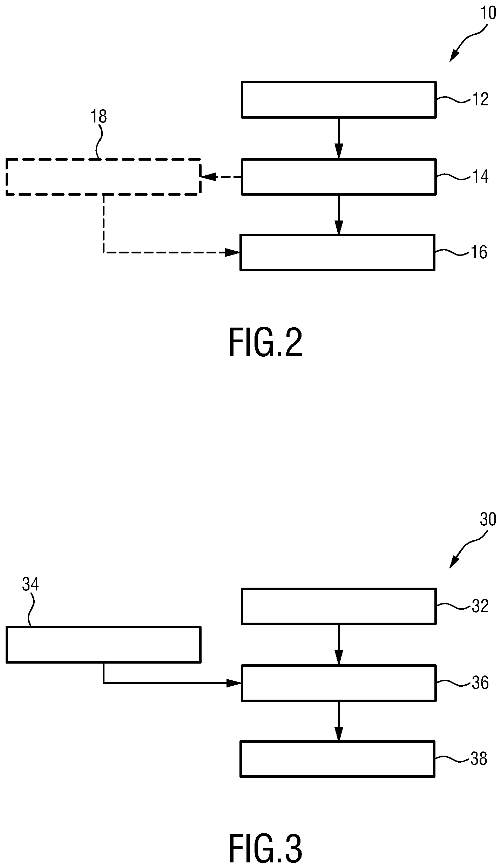

Fig. 2 shows a schematic diagram of an embodiment of a device for estimating a probability of a health state of a patient according to the present invention. The device 10 may e.g. be implemented as or in a processing unit or computer, in software and/or hardware. The device 10 comprises an input unit 12 configured to obtain a plurality of parameter time series, the parameters being related to physiological data of the patient. The parameter time series may comprise any measurable vital sign, such as heart rate, respiration rate, blood pressure, (body) temperature, or oxygen saturation. The input unit 12 may be directly coupled or connected to any one of a user interface configured to obtain user input, particularly physiological data of the patient and/or a plurality of time series extracted from physiological data of the patient, a database comprising physiological data of the patient and/or a plurality of time series extracted from physiological data of the patient, one or more patient sensors configured to measure one or more physiological parameters of the patient, or may obtain (i.e. retrieve or receive) these data from a storage, buffer, network, or bus, etc. The input may thus e.g. be a (wired or wireless) communication interface or data interface, such as a Bluetooth interface, Wi-Fi interface, LAN interface, HDMI interface, direct cable connect, or any other suitable interface allowing signal transfer to the device.

The device 10 further comprises a processor 14 configured to extract the plurality of time series from the physiological data and optionally further to derive other parameter time series from said plurality of time series. The parameter time series may comprise any measurable vital sign, such as heart rate, respiration rate, blood pressure, (body) temperature, or oxygen saturation. The processor 14 is further configured to estimate the probability of the health state of the patient by applying a machine learning algorithm on the plurality of the (originally obtained/extracted or derived) time series of parameters. The machine learning algorithm used by the processor 14 is based on a GAM, a learned non-linear univariate function is applied on the parameter time series (or more particularly, on each feature of the series) and the results of said application are summed up. The probability of the health state is then obtained by applying the standard sigmoid function on said sum.

The processing 14 may be any kind of means configured to process the data obtained and to estimate the probability of the health state of the patient there from. It may be implemented in software and/or hardware, e.g. as a programmed processor or computer or app on a user device such as a smartphone, smartwatch, tablet, laptop, PC, workstation, etc.

The output unit 16 of the device 10 is configured to provide, particularly output, the probability of the health state, particularly to a rendering unit 130 configured to render audible and/or visual information in accordance with the estimated probability of the health state. The output unit 16 may generally be any interface that provides the estimated probability of the health state, e.g. transmits it to another device or provides it for retrieval by another device (e.g. a smartphone, computer, tablet, etc.). It may thus generally be any (wired or wireless) communication or data interface.

Optionally, the device 10 may further comprise an explanation unit 18. The explanation unit 18 is configured to obtain the results of the learned non-linear univariate functions applied to the parameter time series and to order them by rank for identifying the parameters driving the probability, wherein functions of higher rank contribute more to the probability than those of lower rank. Upon identifying the parameters driving the probability of a health status the explanation unit 18 may provide an explanation for the reasons leading to the estimated probability. For example, if parameters related to the temperature of the patient are identified by the explanation unit 18 to drive the patient's probability of infection, the explanation unit may provide said result to the output unit 16 which is configured to provide the probability of infection (or in general health status) together with an explanation relating to the parameters driving said probability.

Given an augmented GAM, i.e. a GAM comprising feature interaction terms, the explanation unit 18 may further be configured to order the learned bivariate functions by rank to identify interactions driving the probability of the health state. Moreover, the explanation unit 18 may analyze the bivariate functions in terms of the type of interaction underlying the aggregation (conjunctive, disjunctive or compensatory).

Fig. 3 shows a schematic diagram of an embodiment of a training device for training a generalized additive model according to the present invention. The training device 30 comprises an input unit 32, a storage unit 34, a learning unit 36 and an output unit 38.

The input unit 32 is configured to obtain training data comprising example parameter time series, wherein each example parameter time series is labelled with a label indicating a health status. The example parameter time series may comprise any measurable vital sign, such as heart rate, respiration rate, blood pressure, (body) temperature, or oxygen saturation. The input unit 32 may be directly coupled or connected to any one of a user interface configured to obtain user input, particularly a plurality of time series extracted from physiological data of the patient, a database comprising a plurality of time series extracted from physiological data of the patient or may obtain (i.e. retrieve or receive) these data from a storage, buffer, network, or bus, etc. The input may e.g. be a (wired or wireless) communication interface or data interface, such as a Bluetooth interface, Wi-Fi interface, LAN interface, HDMI interface, direct cable connect, or any other suitable interface allowing signal transfer to the training device.

In the storage unit 34 of the training device 30 there is stored a generalized additive model (GAM), wherein the GAM is modelled such that the results of an application of a non-linear univariate function (which is to be learned) on a parameter time series are summed up. GAMs are widely used in machine learning and are particularly useful when the relationship between predictors (i.e. z) and the response (i.e. y) is complex and possibly non-linear, and when interpretability of the model is important.

The learning unit 36 is configured to learn the non-linear univariate functions of the GAM by using the obtained training data. Preferably, the non-linear univariate functions are learned using a boosting algorithm. The learning unit 36 may be any kind of means configured to process the training data and to learn the non-linear univariate functions of the GAM there from. It may be implemented in software and/or hardware, e.g. as a programmed processor or computer or app on a user device such as a smartphone, smartwatch, tablet, laptop, PC, workstation, etc.

The training device 30 further comprises an output unit 38 configured to provide (particularly output) the GAM with the learned non-linear univariate functions, particularly to a device 10 for estimating, based on the GAM, a probability of a health state of a patient, particularly a probability of an infection of the patient state. The output unit 38 may generally be any interface that provides the GAM, e.g. transmits it to another device or provides it for retrieval by another device (e.g. a smartphone, computer, tablet, etc.). It may thus generally be any (wired or wireless) communication or data interface.

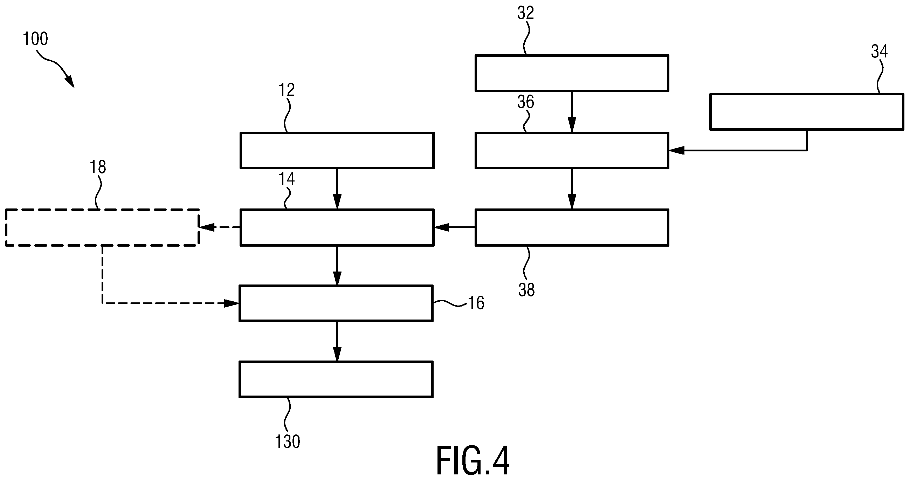

Fig. 4 shows a schematic diagram of a second embodiment of a system according to the present invention. The system 100 comprises the device 10 (comprising an input unit 12, a processor 14, an output unit 16 and optionally an explanation unit 18), the training device 30 (comprising an input unit 32, a storage unit 34, a learning unit 36 and an output unit 38) and a rendering unit 130. The GAM comprising learned non-linear univariate functions is, in this embodiment, is shown to be directly provided to the processor 14. However, to be more precisely it is provided to the input unit 12 which then provides the GAM to the processor 14.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (10) for estimating a probability of a health state of a patient,

   particularly a probability of an infection of the patient, the device (10) comprising
   an input unit (12) configured to obtain physiological data of the patient, the physiological data comprising information about a plurality of time series of physiological parameters of the patient,
   a processor (14) configured to extract the plurality of time series from the physiological data and to apply a machine learning algorithm on the plurality of time series to estimate the probability of the health state of the patient, and
   an output unit (16) configured to provide the probability of the health state,
   wherein the machine learning algorithm is based on a generalized additive model, in which results of an application of a learned non-linear univariate function on the parameter time series are summed up and the probability of the health state is obtained by applying the standard sigmoid function on said sum.

2. The device (10) as claimed in claim 1, wherein in the generalized additive model the sum of the learned non-linear functions on the parameter time series is supplemented by an interaction component, the interaction component comprising a learned bivariate function of two of the learned non-linear univariate functions of the parameter time series, wherein the probability of the health state is obtained by applying the standard sigmoid function on a sum of the sum of the learned non-linear univariate functions and the learned bivariate function.

3. The device (10) as claimed in claim 2, wherein the interaction component comprises a sum of learned bivariate functions of two of the learned non-linear univariate functions of the parameter time series, wherein the probability of the health state is obtained by applying the standard sigmoid function on a sum of the sum of the learned non-linear univariate functions and the sum of the learned bivariate functions.

4. The device (10) as claimed in any one of the preceding claims, further comprising an explanation unit (18) configured to identify parameters driving the probability by ordering the learned non-linear univariate functions of the parameter time series by rank.

5. The device (10) as claimed in claim 4, wherein if the sum of the generalized additive model is supplemented by the interaction component as claimed in claim 2, the explanation unit is further configured to analyze the bivariate function to assess whether the corresponding interaction component is conjunctive, disjunctive or compensatory.

6. The device (10) as claimed in any one of the preceding claims, wherein the device (10) is configured to provide the probability of the health state in real time.

7. A method for estimating a probability of a health state of a patient, particularly a probability of an infection of the patient, the method comprising

   obtaining physiological data of the patient, the physiological data comprising information about a plurality of time series of physiological parameters of the patient,
   extracting the plurality of time series from the physiological data and applying a machine learning algorithm on the plurality of time series to estimate the probability of the health state of the patient, and
   providing the probability of the health state,
   wherein the machine learning algorithm is based on a generalized additive model, in which results of an application of a learned non-linear univariate function on the parameter time series are summed up and the probability of the health state is obtained by applying the standard sigmoid function on said sum.

8. A training device (30) for training a generalized additive model, the training device (20) comprising

   an input unit (32) configured to obtain training data comprising example parameter time series, wherein each example parameter time series is labelled with a label indicating a health status,
   a storage unit (34) configured to store the generalized additive model, wherein in the generalized additive model the results of an application of a non-linear univariate function on the parameter time series are summed up for use in estimating a probability of the health status of a patient,
   a learning unit (36) configured to learn the non-linear univariate functions by using the training data, and
   an output unit (38) configured to provide the generalized additive model with the learned non-linear univariate functions.

9. The training device (30) as claimed in claim 7, wherein the learning unit (26) is configured to supplement the sum of the learned non-linear functions on the parameter time series by an interaction component, the interaction component comprising a bivariate function of two of the learned non-linear univariate functions of the parameter time series, particularly a sum of bivariate functions of two of the learned non-linear univariate functions, and to learn the bivariate function or bivariate functions, wherein the output unit (38) is configured to provide the generalized additive model with the learned bivariate function or bivariate functions.

10. The training device (30) as claimed in claim 8, wherein the learning unit (36) is configured to parametrize the bivariate functions as uninorm functions.

11. The training device (30) as claimed in claim 8 or 9, wherein the learning unit (36) is configured to learn the bivariate functions iteratively.

12. The training device (30) as claimed in claim 11, wherein in each iteration step the learning unit (36) is configured to learn the bivariate function using boosting techniques.

13. A method for training a generalized additive model, the method comprising

   obtaining training data comprising example parameter time series, wherein each example parameter time series is labelled with a label indicating a health state,
   storing the generalized additive model, wherein in the generalized additive model the results of an application of a non-linear univariate function on a parameter time series are summed up for use in estimating a probability of a health status of a patient,
   learning the non-linear univariate functions by using the training data, and

providing the generalized additive model with the learned non-linear univariate functions.

14. A system (100) for estimating a probability of a health state of a patient, the system (100) comprising the device (10) as claimed in claim 1 and a rendering unit (130) configured to render visual and/or audible information in accordance with the estimated probability of the health state of the patient.

15. Computer program comprising program code means for causing a computer to carry out the steps of the methods as claimed in claim 7 and/or 13 when said computer program is carried out on the computer.

FIG.1

10

12

18

14

16

# FIG.2

30

34

32

36

38

# FIG.3

FIG.4

| | Europäisches Patentamt European Patent Office Office européen des brevets | **EUROPEAN SEARCH REPORT** | **Application Number** EP 24 17 9014 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TRAN AN ET AL: "Predicting the Onset of Sepsis Using Vital Signs Data: A Machine Learning Approach", CLINICAL NURSING RESEARCH, [Online] vol. 32, no. 7, 30 September 2023 (2023-09-30), pages 1000-1009, XP093223960, ISSN: 1054-7738, DOI: 10.1177/10547738231183207 [retrieved on 2024-11-13] * Page 1001, Sections "Introduction" and "Objectives" * * Pages 1001-1002, Section "Methodology - Dataset" * * Pages 1002-1003, Section "Methodology - Analysis Plan" * * Pages 1003-1004, Section "Model Training - Explainable Boosting Machine" * * Equation 1 * * Pages 1004-1007, Section "Discussion" * * figure 2 * ----- | 1-15 | INV. G16H50/20 A61B5/00 G16H50/30 G16H50/70 |
| A | FENG TING ET AL: "Machine learning-based clinical decision support for infection risk prediction", FRONTIERS IN MEDICINE, vol. 10, 18 December 2023 (2023-12-18), pages 1-12, XP093223962, ISSN: 2296-858X, DOI: 10.3389/fmed.2023.1213411 * Pages 4-5, Sections "Description of algorithms used" and "Description of model interpretation methods" * * figure 2 * ----- -/-- | 1-15 | TECHNICAL FIELDS SEARCHED (IPC) G16H A61B |

The present search report has been drawn up for all claims

| Place of search | Date of completion of the search | Examiner |
|---|---|---|
| Munich | 14 November 2024 | Bonnet, Clara |

EPO FORM 1503 03.82 (P04C01)

page 1 of 2

**EP 4 657 458 A1**

| | Europäisches Patentamt European Patent Office Office européen des brevets | **EUROPEAN SEARCH REPORT** | **Application Number** EP 24 17 9014 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOSLEHI SAMAD ET AL: "Interpretable generalized neural additive models for mortality prediction of COVID-19 hospitalized patients in Hamadan, Iran", BMC MEDICAL RESEARCH METHODOLOGY, [Online] vol. 22, no. 1, 31 December 2022 (2022-12-31), XP093223965, GB ISSN: 1471-2288, DOI: 10.1186/s12874-022-01827-y Retrieved from the Internet: URL:https://link.springer.com/article/10.1 186/s12874-022-01827-y/fulltext.html> [retrieved on 2024-11-13] * Pages 7-8, Section "Generalized Neural Additive Models" * * figure 3 * ----- | 1-15 | |
| A | Cui Zhicheng ET AL: "A Factored Generalized Additive Model for Clinical Decision Support in the Operating Room", , 4 March 2020 (2020-03-04), pages 343-352, XP093223966, Retrieved from the Internet: URL:https://pmc.ncbi.nlm.nih.gov/articles/ PMC7153157/ [retrieved on 2024-11-13] * Pages 345-346, Section "Methods - Model Algorithm" * * figure 1 * ----- | 1-15 | **TECHNICAL FIELDS SEARCHED (IPC)** |

The present search report has been drawn up for all claims

| Place of search | Date of completion of the search | Examiner |
|---|---|---|
| Munich | 14 November 2024 | Bonnet, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2